(19) **Europäisches Patentamt / European Patent Office / Office européen des brevets**

(11) **EP 4 364 729 A1**

(12) **EUROPEAN PATENT APPLICATION**
published in accordance with Art. 153(4) EPC

(43) Date of publication:
08.05.2024 Bulletin 2024/19

(21) Application number: 22860441.9

(22) Date of filing: 22.08.2022

(51) International Patent Classification (IPC):
$A61K\ 9/70^{(2006.01)}$   $A61K\ 31/4174^{(2006.01)}$
$A61K\ 47/10^{(2017.01)}$   $A61K\ 47/12^{(2006.01)}$
$A61K\ 47/32^{(2006.01)}$   $A61P\ 25/20^{(2006.01)}$

(52) Cooperative Patent Classification (CPC):
A61K 9/70; A61K 31/4174; A61K 47/10;
A61K 47/12; A61K 47/32; A61P 25/20

(86) International application number:
PCT/CN2022/113953

(87) International publication number:
WO 2023/025095 (02.03.2023 Gazette 2023/09)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 23.08.2021 CN 202110969146

(71) Applicant: Yichang Humanwell Pharmaceutical
Co., Ltd.
Yichang, Hubei 443005 (CN)

(72) Inventors:
• LIU, Peng
Yichang, Hubei 443005 (CN)
• LI, Ke
Yichang, Hubei 443005 (CN)
• WU, Youbin
Yichang, Hubei 443005 (CN)
• LIN, Xia
Yichang, Hubei 443005 (CN)
• YUAN, Jing
Yichang, Hubei 443005 (CN)
• LV, Jinliang
Yichang, Hubei 443005 (CN)
• TIAN, Luanyuan
Yichang, Hubei 443005 (CN)
• LI, Lie
Yichang, Hubei 443005 (CN)

(74) Representative: Modiano, Gabriella Diana et al
Modiano & Partners (DE)
Steinsdorfstrasse, 14
80538 München (DE)

(54) **DEXMEDETOMIDINE TRANSDERMAL COMPOSITION, TRANSDERMAL PATCH AND PREPARATION METHOD THEREFOR AND USE THEREOF**

(57)   Disclosed in the present application are a dexmedetomidine transdermal composition, a transdermal patch and a preparation method therefor and the use thereof. The composition comprises dexmedetomidine, propylene glycol, and a metal chelate crosslinking agent, or dexmedetomidine, propylene glycol, a metal chelate crosslinking agent and a pressure-sensitive adhesive. In addition, according to the present application, propylene glycol is added to the dexmedetomidine transdermal composition as a solubilizer, which can reduce the generation of impurities and make the compatibility of raw materials and auxiliary materials better. Moreover, the skeleton structure of the product is constructed using the metal chelate crosslinking agent and the pressure-sensitive adhesive together, so that the adhesion performance is obviously improved, and patients can achieve an excellent fitting feeling. The product of the present application is good in stability and free from skin irritation, the safety of the product when applied to the human body is significantly improved, and a sustained-release effect over 2-5 days can be achieved.

EP 4 364 729 A1

FIG. 3

**Description**

Related application

The present application claims priority to Chinese invention patent application No. 202110969146.7, filed on August 23, 2021 and titled "Dexmedetomidine Transdermal Composition, Transdermal Patch and Preparation Method Therefor and Use Thereof", the entire contents of which are incorporated herein by reference.

Technical Field

The present application relates to, but is not limited to, the field of pharmaceutical preparations, in particular to a dexmedetomidine transdermal composition, a transdermal patch and a preparation method therefor and use thereof.

Background

At present, the only dexmedetomidine marketed domestically and abroad is dexmedetomidine hydrochloride injection, and it can only be used under close supervision of professional medical personnel. It is clinically used for tracheal intubation and sedation during ventilation of surgical patients under general anesthesia. The half-life of dexmedetomidine after injection is only 2 h, and the efficacy duration is short.

The prior art Chinese patent application No. CN201480059798.5 discloses a dexmedetomidine transdermal composition, in which different dexmedetomidine transdermal compositions are tested for an average dexmedetomidine flux as a function of application time. The inventors of the present application have found that the dexmedetomidine transdermal compositions provided in that application have a poor flux, in some embodiments, the transdermal compositions only provide a flux of less than 0.5 ug/cm$^2$*h, and the transdermal diffusion rate reaches its maximum at the 24th hour.

Therefore, there is an urgent need to develop a dexmedetomidine transdermal composition, which can provide a superior transdermal diffusion rate, storage stability, and ease of voluntary administration by patients, with the number of administrations able to be significantly reduced and the duration of action in vivo significantly prolonged.

Summary

In view of this, the present application provides a dexmedetomidine transdermal composition, a transdermal patch and a preparation method therefor and use thereof. This product has a good transdermal diffusion rate and good stability, and can achieve sustained release effect of 2-5 days.

The following is a summary of the subject matter described in detail herein. This summary is not intended to limit the protection scope of the present application.

In an aspect, the present application provides a dexmedetomidine transdermal composition, including dexmedetomidine, propylene glycol, and a metal chelate crosslinking agent;

optionally, in parts by weight, in the composition,

dexmedetomidine 0.30-3.00 parts

propylene glycol 0.40-8.00 parts

metal chelate crosslinking agent 0.30-1.25 parts;

or, the mass ratio of propylene glycol to dexmedetomidine is (4:3)-(8:3).

In a second aspect, the present application provides a dexmedetomidine transdermal composition, including dexmedetomidine, propylene glycol, a metal chelate crosslinking agent, and a pressure-sensitive adhesive;

optionally, in parts by weight, in the composition,

dexmedetomidine 0.30-3.00 parts

propylene glycol 0.40-8.00 parts

metal chelate crosslinking agent 0.30-1.25 parts

pressure-sensitive adhesive 50.00-99.00 parts

and/or, the mass ratio of propylene glycol to dexmedetomidine is (4:3)-(8:3).

In a third aspect, the present application provides a dexmedetomidine transdermal patch, wherein the transdermal patch includes in the following order a backing layer, an adhesive layer, and an anti-adhesion release film layer; and the adhesive layer is formed by the dexmedetomidine transdermal composition of the first aspect or the second aspect.

In a fourth aspect, the present application provides a method for preparing the dexmedetomidine transdermal composition according to the second aspect, including the following steps:

dissolving a metal chelate crosslinking agent in a solvent to obtain a metal chelate crosslinking agent solution;

mixing a pressure-sensitive adhesive with the metal chelate crosslinking agent solution to obtain a blank matrix solution;

mixing dexmedetomidine, propylene glycol, and a solvent to obtain a dexmedetomidine solution; and

mixing the dexmedetomidine solution with the blank matrix solution, followed by stirring, standing, and drying.

In a fifth aspect, the present application provides a method for preparing the dexmedetomidine transdermal patch according to the third aspect, including the following steps:

dissolving a metal chelate crosslinking agent in a solvent to obtain a metal chelate crosslinking agent solution;

mixing a pressure-sensitive adhesive with the metal chelate crosslinking agent solution to obtain a blank matrix solution;

mixing dexmedetomidine, propylene glycol, and a solvent to obtain a dexmedetomidine solution;

mixing the dexmedetomidine solution with the blank matrix solution, followed by stirring and standing, to obtain a drug-containing matrix solution;

coating the drug-containing matrix solution onto an anti-adhesion release film layer, and drying to obtain a composite layer of an adhesive layer and the anti-adhesion release film layer; and

laminating a backing layer onto the adhesive layer.

In a sixth aspect, the present application provides use of the dexmedetomidine transdermal composition or the dexmedetomidine transdermal patch in the preparation of a pharmaceutical preparation for improving a sleep disorder.
In a seventh aspect, the present application provides a method for improving a sleep disorder in a subject, the method including administering the above-mentioned dexmedetomidine transdermal composition or dexmedetomidine transdermal patch to a subject in need thereof.
In the first aspect, the present application provides a dexmedetomidine transdermal composition, including dexmedetomidine, propylene glycol, and a metal chelate crosslinking agent.
In some embodiments of the first aspect, in parts by weight, in the composition,

dexmedetomidine 0.30-3.00 parts

propylene glycol 0.40-8.00 parts

metal chelate crosslinking agent 0.30-1.25 parts.

In some embodiments of the first aspect, in the composition,

dexmedetomidine 0.72-3.00 parts

propylene glycol 1.20-8.00 parts

metal chelate crosslinking agent 0.30-1.25 parts.

In some embodiments of the first aspect, in the composition,

dexmedetomidine 0.72-1.80 parts

propylene glycol 1.20-4.20 parts

metal chelate crosslinking agent 0.30-0.80 parts.

In some embodiments of the first aspect, in the composition,

dexmedetomidine 0.72-1.45 parts

propylene glycol 1.20-4.20 parts

metal chelate crosslinking agent 0.30-0.80 parts.

In some embodiments of the first aspect, the mass ratio of propylene glycol to dexmedetomidine in the composition is (4:3)-(8:3).
In some embodiments of the first aspect, the mass ratio of propylene glycol to dexmedetomidine in the composition is (5:3)-(7:3).
In some embodiments of the first aspect, the mass ratio of propylene glycol to dexmedetomidine in the composition is 5:3.
In some embodiments of the first aspect, the metal chelate crosslinking agent is selected from one or more of aluminum acetylacetonate, zirconium acetylacetonate, titanium acetylacetonate, and polybutyl titanate.
In some embodiments of the first aspect, the metal chelate crosslinking agent is aluminum acetylacetonate or polybutyl titanate.
In some embodiments of the first aspect, the metal chelate crosslinking agent titanium acetylacetonate is titanium (oxy)acetylacetonate or titanium tetraacetylacetonate, or a mixture thereof.
In the second aspect, the present application provides a dexmedetomidine transdermal composition, including dexmedetomidine, propylene glycol, a metal chelate crosslinking agent, and a pressure-sensitive adhesive.
In some embodiments of the second aspect, in parts by weight, in the composition,

dexmedetomidine 0.30-3.00 parts

propylene glycol 0.40-8.00 parts

metal chelate crosslinking agent 0.30-1.25 parts

pressure-sensitive adhesive 50.00-99.00 parts.

In some embodiments of the second aspect, in the composition,

dexmedetomidine 0.30-3.00 parts

propylene glycol 0.40-8.00 parts

metal chelate crosslinking agent 0.30-1.25 parts

pressure-sensitive adhesive 50.00-99.00 parts

the mass ratio of propylene glycol to dexmedetomidine is (4:3)-(8:3).

In some embodiments of the second aspect, the composition includes 0.72-3.00 parts, 0.72-1.80 parts, 0.72-1.45 parts, or 1.00-1.80 parts of dexmedetomidine.

In some embodiments of the second aspect, the mass ratio of the pressure-sensitive adhesive to the metal chelate crosslinking agent in the composition is (70:1)-(310:1).

In some embodiments of the second aspect, in the composition,

dexmedetomidine 0.72-3.00 parts

propylene glycol 1.20-8.00 parts

metal chelate crosslinking agent 0.30-1.25 parts

pressure-sensitive adhesive 87.00-99.00 parts.

In some embodiments of the second aspect, in the composition,

dexmedetomidine 0.72-1.80 parts

propylene glycol 1.20-4.20 parts

metal chelate crosslinking agent 0.30-0.80 parts

pressure-sensitive adhesive 93.00-99.00 parts.

In some embodiments of the second aspect, in the composition,

dexmedetomidine 0.72-1.45 parts

propylene glycol 1.20-4.20 parts

metal chelate crosslinking agent 0.30-0.80 parts

pressure-sensitive adhesive 95.00-99.00 parts.

In some embodiments of the second aspect, the mass ratio of propylene glycol to dexmedetomidine in the composition is (4:3)-(8:3).

In some embodiments of the second aspect, the mass ratio of propylene glycol to dexmedetomidine in the composition is (5:3)-(7:3).

In some embodiments of the second aspect, the mass ratio of propylene glycol to dexmedetomidine in the composition is 5:3.

In some embodiments of the second aspect, the mass ratio of the pressure-sensitive adhesive to the metal chelate crosslinking agent in the composition is (70:1)-(160:1), (160:1)-(220:1), or (220:1)-(310:1).

In some embodiments of the second aspect, the mass ratio of the pressure-sensitive adhesive to the metal chelate crosslinking agent in the composition is (160:1)- (220:1), (160:1)-(210:1), (160:1)-(200:1), or (190:1)-(220:1).

In some embodiments of the second aspect, the mass ratio of the pressure-sensitive adhesive to the metal chelate crosslinking agent in the composition is 160:1, 170:1, 180:1, 190:1, 200:1, 210:1, or 220:1.

In some embodiments of the second aspect, the mass ratio of the pressure-sensitive adhesive to the metal chelate crosslinking agent in the composition is 190:1.

In some embodiments of the second aspect, the composition includes 0.30-0.70 parts of the metal chelate crosslinking agent.

In some embodiments of the second aspect, the composition includes 0.40-0.60 parts of the metal chelate crosslinking agent.

In some embodiments of the second aspect, the composition includes 0.50 parts of the metal chelate crosslinking agent.

In some embodiments of the second aspect, the metal chelate crosslinking agent is selected from one or more of aluminum acetylacetonate, zirconium acetylacetonate, titanium acetylacetonate, and polybutyl titanate.

In some embodiments of the second aspect, the metal chelate crosslinking agent is aluminum acetylacetonate or polybutyl titanate.

In some embodiments of the second aspect, the metal chelate crosslinking agent titanium acetylacetonate is titanium (oxy)acetylacetonate or titanium tetraacetylacetonate, or a mixture thereof.

In some embodiments of the second aspect, the pressure-sensitive adhesive is selected from one or more of an acrylate pressure-sensitive adhesive, a polyisobutylene pressure-sensitive adhesive, a silicone pressure-sensitive adhesive, a styrene-isoprene-styrene hot melt pressure-sensitive adhesive (SIS type), and a vinyl acetate copolymer.

In some embodiments of the second aspect, the acrylate pressure-sensitive adhesive is Duro-Tak 387-2510 or DURO-TAK 387-2287.

In the third aspect, the present application provides a dexmedetomidine transdermal patch, wherein the transdermal patch includes in the following order a backing layer, an adhesive layer, and an anti-adhesion release film layer; and the adhesive layer is formed by the dexmedetomidine transdermal composition described above.

In some embodiments of the third aspect, the adhesive layer has a thickness of 25 μm-100 μm.

In the fourth aspect, the present application provides a method for preparing the dexmedetomidine transdermal composition described above, including the following steps:

dissolving a metal chelate crosslinking agent in a solvent to obtain a metal chelate crosslinking agent solution;

mixing a pressure-sensitive adhesive with the metal chelate crosslinking agent solution to obtain a blank matrix solution;

mixing dexmedetomidine, propylene glycol, and a solvent to obtain a dexmedetomidine solution; and

mixing the dexmedetomidine solution with the blank matrix solution, followed by stirring, standing, and drying.

In some embodiments of the fourth aspect, the solvent is selected from one of anhydrous ethanol and n-heptane, or a mixture thereof.

In the fifth aspect, the present application provides a method for preparing the dexmedetomidine transdermal patch described above, including the following steps:

dissolving a metal chelate crosslinking agent in a solvent to obtain a metal chelate crosslinking agent solution;

mixing a pressure-sensitive adhesive with the metal chelate crosslinking agent solution to obtain a blank matrix solution;

mixing dexmedetomidine, propylene glycol, and a solvent to obtain a dexmedetomidine solution;

mixing the dexmedetomidine solution with the blank matrix solution, followed by stirring and standing, to obtain a drug-containing matrix solution;

coating the drug-containing matrix solution onto an anti-adhesion release film layer, and drying to obtain a composite layer of an adhesive layer and the anti-adhesion release film layer; and

laminating a backing layer onto the adhesive layer.

In some embodiments of the fifth aspect, the solvent is selected from one of anhydrous ethanol and n-heptane, or a mixture thereof.

In the sixth aspect, the present application provides use of the dexmedetomidine transdermal composition or the dexmedetomidine transdermal patch in the preparation of a pharmaceutical preparation for improving a sleep disorder.

In some embodiments of the sixth aspect, the sleep disorder is one or more of a perioperative sleep disorder, a senile sleep disorder, and a traumatic sleep disorder.

In the seventh aspect, the present application provides a method for improving a sleep disorder in a subject, the method including administering the above-mentioned dexmedetomidine transdermal composition or dexmedetomidine transdermal patch to a subject in need thereof.

In some embodiments of the seventh aspect, the sleep disorder is one or more of a perioperative sleep disorder, a senile sleep disorder, and a traumatic sleep disorder.

In the present application, by creatively adding a specific content of propylene glycol to a dexmedetomidine composition, making the mass ratio of propylene glycol to the main drug dexmedetomidine (4:3)-(8:3), and selecting a metal chelate crosslinking agent and a pressure-sensitive adhesive as the skeleton structure of dexmedetomidine, the dexmedetomidine transdermal composition of the present application has a high in vitro diffusion rate and stable product quality; and compared with the commercial dexmedetomidine hydrochloride injection, it is convenient to administer, and can realize sustained release for 2-5 days, ensuring the sleep quality of patients after use.

Brief Description of Drawings

The accompanying drawings are intended to provide a further understanding of the examples of the present application and form a part of the specification. Together with the following specific embodiments, they are used to explain the examples of the present application and do not form a limitation on the examples of the present application.

FIG. 1 is a schematic diagram of preparation of a dexmedetomidine transdermal patch according to the present application.

FIG. 2 is a schematic structural diagram of a dexmedetomidine transdermal patch according to the present application; wherein A: a side view of the dexmedetomidine transdermal patch; and B: a top view of the dexmedetomidine transdermal patch.

FIG. 3 is a diagram of in vitro transdermal diffusion curves of dexmedetomidine transdermal patches according to the present application with different thicknesses.

FIG. 4 is a diagram of crystallization of a dexmedetomidine transdermal composition according to the present application after 6 months of storage; wherein A is 10×imaging and B is 40× imaging.

FIG. 5 is a line chart of mouse voluntary activity data for use of a dexmedetomidine transdermal composition according to the present application in sleep improvement.

FIG. 6 is a graph of trend of 72 h rat sleep-wake structural cumulative time for use of a dexmedetomidine transdermal composition according to the present application in sleep improvement; A: a graph of Wake time cumulative trend of each group within each time node in the rat sleep structure within 72 h after administration of a patch; B: a graph of NREM time cumulative trend of each group in each time node within 72 h after administration of a patch; C: a graph of REM time cumulative trend of each group in each time node within 72 h after administration of a patch; N=8.

FIG. 7 shows comparative curves of per unit area transdermal diffusion rate of Example 4 and Example 7 of the present application, and Comparative Example 1.

Detailed Description

In the present application, some terms are defined as follows:

NREM sleep: non-rapid eye movement sleep, characterized by slowing down of brain and eye activity, which belongs to the main stage of deep sleep and metabolism.
REM sleep: rapid eye movement sleep, characterized by that brain activity has not yet stopped completely, which belongs to the stage of light sleep and dreaming.

In an embodiment of the present application, in a dexmedetomidine composition provided by the present application, the calculation method of each component is as follows:

$$\% \text{ by weight X } (\%) = X / (A+B+C+D*d)*100\%$$

wherein A is the weight of dexmedetomidine, B is the weight of propylene glycol, C is the weight of the metal chelate crosslinking agent, D is the weight of the pressure-sensitive adhesive, and d is the solid content corresponding to the type of the pressure-sensitive adhesive used in the composition; wherein X may be optionally A, B, C, or D*d.

In an embodiment of the present application, the present application provides a dexmedetomidine transdermal composition, including dexmedetomidine, propylene glycol, a metal chelate crosslinking agent, and a pressure-sensitive adhesive, wherein the content of dexmedetomidine is 0.30-3.00 parts, and the content ratio of propylene glycol to dexmedetomidine is (4:3)-(8:3).

In an embodiment of the present application, the dexmedetomidine transdermal composition provided by the present application includes 0.72-1.45 parts or 1.00-1.80 parts of dexmedetomidine; preferably, 0.72-1.00 parts, 1.00-1.45 parts, or 1.45-1.80 parts of dexmedetomidine.

In an embodiment of the present application, the dexmedetomidine transdermal composition provided by the present application includes 0.72 parts, 1.00 parts, 1.45 parts, or 1.80 parts of dexmedetomidine; more preferably, 1.00 part of dexmedetomidine.

In an embodiment of the present application, in the dexmedetomidine transdermal composition provided by the present application, the content ratio of propylene glycol to dexmedetomidine is 4:3, 5:3, 6:3, 7:3, or 8:3; preferably, the content ratio of propylene glycol to dexmedetomidine is (5:3)-(7:3); and more preferably, the content ratio of propylene glycol to dexmedetomidine is 5:3.

In an embodiment of the present application, the dexmedetomidine transdermal composition provided by the present application includes 0.40-8.00 parts, 1.20-4.20 parts, 1.31-2.42 parts, 1.44-2.42 parts, 1.44-1.67 parts, 1.44-4.20 parts, 1.44-8.00 parts, 1.31-1.67 parts, or 1.67-2.42 parts of propylene glycol; preferably, 1.20-4.20 parts of propylene glycol.

In an embodiment of the present application, the dexmedetomidine transdermal composition provided by the present application includes 0.4 parts, 1.2 parts, 1.31 parts, 1.44 parts, 1.67 parts, 2.42 parts, 4.20 parts, or 8.00 parts of propylene glycol; preferably, 1.67 parts of propylene glycol.

In an embodiment of the present application, in the dexmedetomidine transdermal composition provided by the present application, the mass ratio of the pressure-sensitive adhesive to the metal chelate crosslinking agent is (70:1)-(160:1), (160:1)-(220:1), or (220:1)-(310:1); preferably, the mass ratio of the pressure-sensitive adhesive to the metal chelate crosslinking agent is (160:1)-(190:1) or (190:1)-(220:1); and more preferably, the mass ratio of the pressure-sensitive adhesive to the metal chelate crosslinking agent is 190:1.

In an embodiment of the present application, in the dexmedetomidine transdermal composition provided by the present application, the metal chelate crosslinking agent is selected from one or more of aluminum acetylacetonate, zirconium acetylacetonate, titanium acetylacetonate, and polybutyl titanate.

In an embodiment of the present application, in the dexmedetomidine transdermal composition provided by the present application, the metal chelate crosslinking agent titanium acetylacetonate is selected from one or both of titanium (oxy) acetylacetonate and titanium tetraacetylacetonate.

In an embodiment of the present application, in the dexmedetomidine transdermal composition provided by the present application, the content range of the metal chelate crosslinking agent is 0.30-1.25 parts, 0.30-0.80 parts, 0.30-0.60 parts, 0.45-0.60 parts, 0.45-0.80 parts, 0.45-1.25 parts, or 0.30-0.50 parts; preferably, the content range of the metal chelate crosslinking agent is 0.30-0.80 parts.

In an embodiment of the present application, in the dexmedetomidine transdermal composition provided by the present application, the content of the metal chelate crosslinking agent is 0.30 parts, 0.45 parts, 0.50 parts, 0.60 parts, 0.80 parts, or 1.25 parts; preferably, the content of the metal chelate crosslinking agent is 0.5 parts.

In an embodiment of the present application, in the dexmedetomidine transdermal composition provided by the present application, the pressure-sensitive adhesive is selected from one or more of an acrylate pressure-sensitive adhesive, a polyisobutylene pressure-sensitive adhesive, a silicone pressure-sensitive adhesive, a styrene-isoprene-styrene hot melt pressure-sensitive adhesive (SIS type), and a vinyl acetate copolymer.

The acrylate pressure-sensitive adhesive is one of the following types of pressure-sensitive adhesives: DURO-TAK 387-2287, DURO-TAK 387-2510, DURO-TAK 387-2516, DURO-TAK 87-235A, DURO-TAK 387-2353, DURO-TAK 387-2852, DURO-TAK 387-2051, DURO-TAK 387-2052, DURO-TAK 387-2054, DURO-TAK 87-4287, DURO-TAK 87-6908, and DURO-TAK 87-267.

In an embodiment of the present application, in the dexmedetomidine transdermal composition provided by the present application, the content ratio of the pressure-sensitive adhesive to the metal chelate crosslinking agent is (70:1)-(310:1), (70:1)-(220:1), (70:1)-(200:1), (110:1)-(310:1), (110:1)-(220:1), (110:1)-(200:1), (150:1)-(310:1), (150:1)-(220:1), (150:1)-(200:1), (180:1)-(310:1), (180:1)-(220: 1), or (180:1)-(200:1).

In an embodiment of the present application, the dexmedetomidine transdermal composition provided by the present application includes 0.72-1.80 parts of dexmedetomidine, 0.30-0.80 parts of a metal chelate crosslinking agent, 93.00-99.00 parts of an acrylate pressure-sensitive adhesive, and propylene glycol, wherein the content ratio of propylene glycol to dexmedetomidine is (4:3)-(8:3).

In an embodiment of the present application, in the dexmedetomidine transdermal composition provided by the present application, the metal chelate crosslinking agent is polybutyl titanate or aluminum acetylacetonate.

In an embodiment of the present application, in the dexmedetomidine transdermal composition provided by the present application, the acrylate pressure-sensitive adhesive is Duro-Tak 387-2510 or DURO-TAK 387-2287.

In an embodiment of the present application, provided is the use of the dexmedetomidine transdermal composition provided by the present application in the preparation of a transdermal patch.

In an embodiment of the present application, the dexmedetomidine transdermal patch provided by the present application includes the dexmedetomidine transdermal composition.

In an embodiment of the present application, in the dexmedetomidine transdermal patch provided by the present application, the dexmedetomidine transdermal composition forms an adhesive layer of the transdermal patch.

In an embodiment of the present application, in the dexmedetomidine transdermal patch provided by the present application, the thickness of the adhesive layer is 25 $\mu$m-100 $\mu$m.

In an embodiment of the present application, the dexmedetomidine transdermal patch provided by the present application includes a backing layer, an anti-adhesion release film layer, and an adhesive layer disposed between the backing layer and the anti-adhesion release film layer; the adhesive layer includes 0.72-1.80 parts of dexmedetomidine, 0.30-0.80 parts of polybutyl titanate, 93.00-99.00 parts of an acrylate pressure-sensitive adhesive, and propylene glycol; the content ratio of propylene glycol to dexmedetomidine is (4:3)-(8:3), and the acrylate pressure-sensitive adhesive is Duro-Tak 387-2510 or DURO-TAK 387-2287.

The material of the backing layer is selected from one or more of an aluminum-polyester film, a polyester-polyethylene composite film, a polyethylene-aluminum-polyester /ethylene-vinyl acetate composite film, a multilayer polyester film, and a polyester-ethylene vinyl acetate composite film.

The material of the release film is selected from one or more of a silicified polyester thin film, a fluoropolymer coated polyester thin film, an aluminum foil-silicone grease composite, a silicified aluminum foil, and silicon paper.

In an embodiment of the present application, the method for preparing a dexmetotropine transdermal patch provided by the present application includes the following steps:

(a) dissolving a metal chelate crosslinking agent in a solvent to obtain a metal chelate crosslinking agent solution;

(b) mixing a pressure-sensitive adhesive with the metal chelate crosslinking agent solution to obtain a blank matrix solution;

(c) dissolving propylene glycol, a solvent, and dexmedetomidine, then adding them into the blank matrix solution, and mixing to obtain a drug-containing matrix solution;

(d) coating the drug-containing matrix solution onto a release film and drying to obtain a composite layer of an adhesive layer and an anti-adhesion release film layer; and

(e) laminating a backing film onto the adhesive layer to obtain a dexmedetomidine transdermal patch.

The solvent includes anhydrous ethanol and n-heptane.

In an embodiment of the present application, the present application provides use of the dexmedetomidine transdermal composition or dexmedetomidine transdermal patch in improving a sleep disorder, the sleep disorder including a perioperative sleep disorder, a senile sleep disorder, and a traumatic sleep disorder.

In an embodiment of the present application, the dexmedetomidine transdermal composition or dexmedetomidine transdermal patch provided by the present application can achieve slow release for 2-5 days.

The propylene glycol used in the present application can play a very good effect of assisting the dissolution of dexmedetomidine at a low concentration, can improve the solubility of dexmedetomidine in a skeleton material, and has good compatibility with the bulk drug dexmedetomidine.

The metal chelate crosslinking agent and the pressure-sensitive adhesive used in the present application are jointly used as a skeleton structure of the transdermal composition of the present application, synergistically adjust the adhesion performance of the transdermal composition, and also provide a drug-carrying matrix for dexmedetomidine.

The release film used in the present application can be well compatible with the pressure-sensitive adhesive, which ensures that the release film can be firmly adhered to the surface of the pressure-sensitive adhesive, can be easily peeled off from the adhesive layer when it is torn off, and can withstand the erosion of a variety of raw and auxiliary materials and solvents during the preparation and storage of the transdermal patch.

The backing film used in the present application has good flexibility, has a firm and smooth texture, has suitable peel strength, and also has good compatibility with the raw and auxiliary materials of the transdermal patch of the present application.

The solvent used in the present application includes anhydrous ethanol and n-heptane, which is used for dissolving the bulk drug dexmedetomidine and/or the metal chelating agent crosslinking agent, and has the advantages of being non-toxic, non-irritating, and having good compatibility with raw and auxiliary materials.

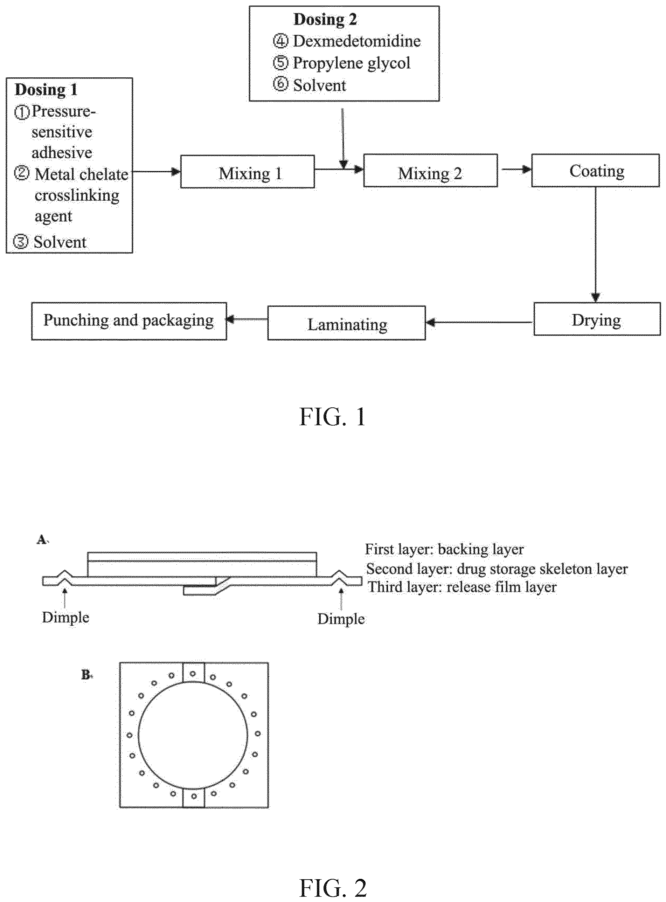

The preparation process of the dexmedetomidine transdermal patch of the present application is shown in FIG. 1, and the detailed structure of the transdermal patch is shown in FIG. 2 (A: a side view of the dexmedetomidine transdermal patch; B: a top view of the dexmedetomidine transdermal patch);
wherein the release film is overlapped, and forms the current anti-adhesion layer with the dimples punched at the periphery of the patch to further reduce the risk of cold flow.

The use or administration method of the dexmedetomidine transdermal patch provided by the present application is: sticking the dexmedetomidine transdermal patch to the unstimulated and unirradiated smooth and flat skin surface of the torso or upper arm of a human body; and if the administration were to be interrupted, the transdermal patch can just be torn off.

The reagents or instruments used in the present application are all commercially available. Instruments: HS-3 vertical mixing instrument, TB-04D precision coater for experiments, DGF302-BN electric drying oven, PL-3002-IC electronic balance, KQ-500VDE ultrasonic cleaning machine, MDC-25SX digital micrometer, XMTD-204 constant temperature magnetic stirrer, Franz transdermal diffusion instrument TK-24BL, GHP-9160 constant temperature incubator, and WD-A drug stability tester.

**Example 1 Study on compatibility stability of dexmedetomidine with solubilizer**

1. Sample preparation

Experimental groups of "dexmedetomidine + levulinic acid", "dexmedetomidine + oleic acid", "dexmedetomidine + laurocapram", "dexmedetomidine + isopropyl myristate", and "dexmedetomidine + propylene glycol" were set, and experimental samples were prepared according to the ratio of dexmedetomidine:solubilizer=1:10.

2. Experiment of compatibility of raw and auxiliary materials

The above experimental samples were prepared into five subgroups, which were a control group, a high temperature group, a high humidity group, a light irradiation group, and an acceleration group. The corresponding conditions are shown in Table 1 below. The sampling time points of high temperature, high humidity, light irradiation, and acceleration were Day 0, Day 5, and Day 10, for investigating the impurity content of the mixture of dexmedetomidine and solubilizer in the present application under different conditions.

Evaluation criteria: maximum unknown single impurity (%, ≤0.5%) total impurity (%, ≤2.0%)

Table 1 Sample testing conditions for study on compatibility stability of raw and auxiliary materials

| Sample name | Condition |
|---|---|
| Control group | / |
| High temperature group | 60°C |
| High humidity group | 25°C/95% RH |
| Light irradiation group | 4500±500lx |
| Acceleration group | 40°C/75% RH |

3. Experimental results

Table 2 Results of experiment of raw and auxiliary material compatibility stability of dexmedetomidine and levulinic acid

| Item | Control group | High temperature group | | High humidity group | | Light irradiation group | | Acceleration group | |
|---|---|---|---|---|---|---|---|---|---|
| | | Day 5 | Day 10 | Day 5 | Day 10 | Day 5 | Day 10 | Day 5 | Day 10 |
| Maximum single impurity (%) | ND | 1.61% | 1.99% | 0.10% | 0.06% | 0.47% | 0.94% | 0.45% | 0.60% |
| Total impurity (%) | ND | 5.25% | 5.53% | 0.40% | 0.33% | 2.72% | 3.35% | 0.70% | 1.85% |

wherein ND means non-detected.

It can be seen from the above table that for the "bulk drug + levulinic acid" group, under the high temperature condition, the maximum single impurity content is 1.99%, and the maximum total impurity content is 5.53%; under the light irradiation condition, the maximum single impurity content is 0.94%, and the maximum total impurity content is 3.35%; and under the acceleration condition, the maximum single impurity content is 0.60%, and the maximum total impurity content is 1.85%. Under the above investigation conditions, the impurity content of "bulk drug + levulinic acid" group exceeds the standard limit, so the raw and auxiliary material compatibility stability of dexmedetomidine and levulinic acid is poor.

Table 3 Results of experiment of raw and auxiliary material compatibility stability of dexmedetomidine and oleic acid

| Item | Control group | High temperature group | | High humidity group | | Light irradiation group | | Acceleration group | |
|---|---|---|---|---|---|---|---|---|---|
| | | Day 5 | Day 10 | Day 5 | Day 10 | Day 5 | Day 10 | Day 5 | Day 10 |
| Maximum single impurity (%) | 0.01% | 0.07% | 1.22% | 0.12% | 0.22% | 0.21% | 0.64% | 0.21% | 0.88% |
| Total impurity (%) | 0.03% | 2.33% | 4.76% | 0.14% | 0.34% | 2.23% | 2.68% | 1.03% | 2.12% |

It can be seen from the above table that for the "bulk drug + oleic acid" group, under the high temperature condition, the maximum single impurity content is 1.22%, and the maximum total impurity content is 4.76%; under the light irradiation condition, the maximum single impurity content is 0.64%, and the maximum total impurity content is 2.68%; and under the acceleration condition, the maximum single impurity content is 0.88%, and the maximum total impurity content is 2.12%. Under the above investigation conditions, the impurity content of "bulk drug + oleic acid" group exceeds the standard limit, so the raw and auxiliary material compatibility stability of dexmedetomidine and oleic acid is poor.

Table 4 Results of experiment of raw and auxiliary material compatibility stability of dexmedetomidine and laurocapram

| Item | Control group | High temperature group | | High humidity group | | Light irradiation group | | Acceleration group | |
|---|---|---|---|---|---|---|---|---|---|
| | | Day 5 | Day 10 | Day 5 | Day 10 | Day 5 | Day 10 | Day 5 | Day 10 |
| Maximum single impurity (%) | 0.02% | 2.04% | 2.21% | 0.14% | 0.26% | 0.92% | 0.96% | 0.42% | 0.97% |
| Total impurity (%) | 0.06% | 3.44% | 4.29% | 0.22% | 1.82% | 2.36% | 3.54% | 3.86% | 3.67% |

It can be seen from the above table that for the "bulk drug + laurocapram" group, under the high temperature condition, the maximum single impurity content is 2.21%, and the maximum total impurity content is 4.29%; under the light irradiation condition, the maximum single impurity content is 0.96%, and the maximum total impurity content is 3.54%; and under the acceleration condition, the maximum single impurity content is 0.97%, and the maximum total impurity content is 3.86%. Under the above investigation conditions, the impurity content of "bulk drug + laurocapram" group exceeds the standard limit, so the raw and auxiliary material compatibility stability of dexmedetomidine and laurocapram is poor.

Table 5 Results of experiment of raw and auxiliary material compatibility stability of dexmedetomidine and isopropyl myristate

| Item | Control group | High temperature group | | High humidity group | | Light irradiation group | | Acceleration group | |
|---|---|---|---|---|---|---|---|---|---|
| | | Day 5 | Day 10 | Day 5 | Day 10 | Day 5 | Day 10 | Day 5 | Day 10 |
| Maximum single impurity (%) | ND | 0.09% | 1.03% | 0.15% | 0.18% | 0.17% | 0.72% | 0.17% | 0.72% |
| Total impurity (%) | ND | 2.54% | 3.36% | 0.35% | 1.68% | 2.46% | 4.79% | 2.98% | 4.32% |

It can be seen from the above table that for the "bulk drug + isopropyl myristate" group, under the high temperature condition, the maximum single impurity content is 1.03%, and the maximum total impurity content is 3.36%; under the light irradiation condition, the maximum single impurity content is 0.72%, and the maximum total impurity content is 4.79%; and under the acceleration condition, the maximum single impurity content is 0.72%, and the maximum total impurity content is 4.32%. Under the above investigation conditions, the impurity content of "bulk drug + isopropyl myristate" group exceeds the standard limit, so the raw and auxiliary material compatibility stability of dexmedetomidine and isopropyl myristate is poor.

Table 6 Results of experiment of raw and auxiliary material compatibility stability of dexmedetomidine and propylene glycol

| Item | Control group | High temperature group | | High humidity group | | Light irradiation group | | Acceleration group | |
|---|---|---|---|---|---|---|---|---|---|
| | | Day 5 | Day 10 | Day 5 | Day 10 | Day 5 | Day 10 | Day 5 | Day 10 |
| Maximum single impurity (%) | 0.02% | 0.02% | 0.03% | 0.02% | 0.03% | 0.02% | 0.04% | 0.02% | 0.04% |
| Total impurity (%) | 0.05% | 0.04% | 0.03% | 0.04% | 0.05% | 0.05% | 0.05% | 0.04% | 0.05% |

It can be seen from the above table that for the "bulk drug + propylene glycol" group, under all the investigation conditions, the maximum single impurity content is 0.03%, and the maximum total impurity content is 0.05%. Therefore, the raw and auxiliary material compatibility stability of dexmedetomidine and propylene glycol is very good.

To sum up, it can be seen from Tables 2-6 that "dexmedetomidine + levulinic acid", "dexmedetomidine + oleic acid", "dexmedetomidine + laurocapram", and "dexmedetomidine + isopropyl myristate" groups all produce large amounts of impurities under the high temperature and light irradiation conditions, indicating that the compatibility stability of the above auxiliary materials with the bulk drug dexmedetomidine is poor, while for the "bulk drug + propylene glycol" group, under all the investigation conditions, the maximum single impurity content is 0.03%, and the maximum total large impurity content is 0.05%, indicating that the compatibility stability of the auxiliary material with the bulk drug is very good. Therefore, propylene glycol was selected as the solubilizer of the transdermal composition of the present application, and the addition of propylene glycol can reduce the generation of impurities in the later stage.

**Experimental Example 2 Investigation on the amount of propylene glycol**

**1. Prescription 1-transdermal patch**

Dexmedetomidine 0.08 g

Acrylate pressure-sensitive adhesive 20.12 g

Aluminum acetylacetonate 0.05 g

Propylene glycol 0.16 g

Anhydrous ethanol 3.41 g

N-heptane 1.34 g

The model of the acrylate pressure-sensitive adhesive is Duro-Tak 387-2287, with a solid content of 53.8%.

The transdermal patch includes a backing layer, an adhesive layer, and an anti-adhesion release film layer, and the preparation method of the transdermal patch specifically includes the following steps:

(1) preparation of an aluminum acetylacetonate solution: weighing and adding 0.05 g aluminum acetylacetonate, 1.01 g anhydrous ethanol, and 1.34 g n-heptane into a 40 mL scientific vial, and mixing them well for later use;

(2) preparation of a blank matrix: weighing 20.12 g acrylate pressure-sensitive adhesive and the prepared aluminum acetylacetonate solution, and sealing them;

(3) mixing: placing the scientific vial containing the blank matrix on a vertical mixer, adjusting the rotating speed to 20 rpm, and stirring for 4 h;

(4) preparation of a drug-containing matrix: weighing 0.08 g dexmedetomidine, adding 0.16 g propylene glycol and 2.24 g anhydrous ethanol, stirring until dexmedetomidine was completely dissolved, then adding the mixture solution into the blank matrix which was stirred for 4 h, weighing 0.16 g anhydrous ethanol to wash the emptied vial, adding it into the drug-containing matrix, mixing, and sealing;

(5) mixing: placing the scientific vial containing the drug-containing matrix on the vertical mixer, adjusting the rotating speed to 20 rpm, stirring for 6 h and then stop, and standing overnight;

(6) coating: coating the drug-containing matrix after standing overnight onto a release film Scotchpak 1022, adjusting the thickness of the coating blade until the thickness of the drug-containing layer of the final transdermal patch was 25 μm;

(7) drying: placing it in an oven, adjusting the temperature to 70 °C, and taking it out 20 min later;

(8) coating with a film: laminating the backing film Scotchpak 9722 onto the drug-containing adhesive layer; and

(9) punching: punching the resultant product into a round patch with a diameter (D=30 mm), and packaging the same.

All anhydrous ethanol and n-heptane will be removed during the preparation process, and will not appear in the final product.

## 2. Experimental grouping

Different amounts of propylene glycol were selected for propylene glycol screening for prescription optimization. Specifically, the dosing weight ratio of propylene glycol to dexmedetomidine was 3:3, 4:3, 5:3, 6:3, 7:3, 8:3, 9:3, and a blank group was set (no propylene glycol was added in prescription 1).

## 3. Detection of related substances

The following 8 groups of samples (n=6) were detected for impurities, and the results of impurities of related substances are shown in Table 7.

Table 7 Results of impurities of related substances for amount screening of propylene glycol

| Group | Propylene glycol: dexmedetomidine | Single impurity (%) | Total impurity (%) |
|---|---|---|---|
| 1 | 0:3 | 0.5 | 0.8 |
| 2 | 3:3 | 0.5 | 0.7 |
| 3 | 4:3 | 0.5 | 0.8 |
| 4 | 5:3 | 0.5 | 0.8 |
| 5 | 6:3 | 0.4 | 0.7 |
| 6 | 7:3 | 0.4 | 0.7 |
| 7 | 8:3 | 0.4 | 0.8 |
| 8 | 9:3 | 0.5 | 0.8 |

As can be seen from the above table, the amount of propylene glycol has no obvious effect on the related substances of the transdermal patch in this example.

## 4. Test of adhesion performance

Eight groups of samples (n=6) were subjected to amount screening of propylene glycol. The eight groups of samples were pasted onto the smooth skin of the back of hands of eight volunteers, and were peeled off 30 min later. Whether there was adhesive residue on the skin and whether pain was caused were observed to investigate the adhesion performance of the transdermal patch of the present application.

Scoring indexes of adhesion performance:

1: weak in vivo adhesion performance;

2: slight residue of adhesive edge, good adhesive performance;

3: no residue of adhesive edge, good adhesive performance; and

4: causing pain upon removal, including keratinized skin abrasions.

Results of the experiment of solubility of dexmedetomidine and adhesion of transdermal patch are shown in Table 8.

Table 8 Results of test of amount screening of propylene glycol

| Group | Propylene glycol: dexmedetomidine | Evaluation of in vitro adhesion performance |
|---|---|---|
| 1 | 0:3 | 1 |
| 2 | 3:3 | 1 |
| 3 | 4:3 | 2 |
| 4 | 5:3 | 3 |
| 5 | 6:3 | 3 |
| 6 | 7:3 | 3 |
| 7 | 8:3 | 4 |
| 8 | 9:3 | 4 |

As can be seen from the above table, when the transdermal patch does not contain propylene glycol and when the dosing ratio of propylene glycol to dexmedetomidine is 3:3, the adhesion performance of the samples is weak; when the mass ratio of propylene glycol to dexmedetomidine reaches 4:3 and above, the adhesion performance of the samples is good; however, when the dosing ratio exceeds 8:3, some samples cause pain when torn off; when the dosing ratio ranges from (5:3) to (7:3), the adhesion performance of the patch in this example on the skin surface is better; and when the dosing ratio is 5:3, the adhesion performance of the transdermal patch in this example on the skin surface is the best, and none of the samples has residues on the skin surface and cause pain when torn off.

**Experimental Example 3 Investigation on the amount of metal chelate crosslinking agent**

1. Prescription preparation

(1) Prescription 2

Dexmedetomidine 0.18 g

Acrylate pressure-sensitive adhesive 41.32 g

Polybutyl titanate 0.12 g

Propylene glycol 0.30 g

Anhydrous ethanol 5.70 g

N-heptane 6.19 g

The model of the acrylate pressure-sensitive adhesive was Duro-Tak 387-2287, with a solid content of 53.8%.

The preparation method of Prescription 2 was the same as that of Prescription 1, except that Scotchpak 9754 backing film was laminated onto the drug-containing adhesive layer.

The amounts and contents of dexmedetomidine and propylene glycol of prescription 2 were unchanged, and the amount and content of the metal chelate crosslinking agent-polybutyl titanate were adjusted to obtain Prescription 2' (0.07 g, about 0.30 parts), Prescription 2" (0.09 g, about 0.40 parts), Prescription 2‴ (0.14 g, about 0.60 parts), and Prescription 2⁗ (0.16 g, about 0.70 parts), and the acrylate pressure-sensitive adhesives of corresponding prescriptions were adaptively adjusted (in Prescriptions 2'-2⁗, the amounts of the acrylate pressure-sensitive adhesives were 41.33 g, 40.91 g, 40.49 g, and 41.37 g respectively).

(2) Prescription 3

Dexmedetomidine 0.18 g

Acrylate pressure-sensitive adhesive 41.32 g

Aluminum acetylacetonate 0.12 g

Propylene glycol 0.30 g

Anhydrous ethanol 5.70 g

N-heptane 6.19 g

The model of the acrylate pressure-sensitive adhesive was Duro-Tak 387-2287; and the preparation method was the same as that of Prescription 2.

The amounts and contents of dexmedetomidine and propylene glycol were unchanged, and the amount and content of the metal chelate crosslinking agent were adjusted to obtain Prescription 3' (0.07 g, about 0.30 parts), Prescription 3" (0.09 g, about 0.40 parts), Prescription 3‴ (0.14 g, about 0.60 parts), and Prescription 3⁗ (0.16 g, about 0.70 parts), and the acrylate pressure-sensitive adhesives of corresponding prescriptions were adaptively adjusted (in Prescriptions 3'-3⁗, the amounts of the acrylate pressure-sensitive adhesives were 41.33 g, 40.91 g, 40.49 g, and 41.37 g respectively).

(3) Prescription 4

Dexmedetomidine 0.18 g

Acrylate pressure-sensitive adhesive 41.32 g

Zirconium acetylacetonate 0.12 g

Propylene glycol 0.30 g

Anhydrous ethanol 5.70 g

N-heptane 6.19 g

The model of the acrylate pressure-sensitive adhesive was Duro-Tak 387-2287; and the preparation method was the same as that of Prescription 2.

The amounts and contents of dexmedetomidine and propylene glycol were unchanged, and the amount and content of the metal chelate crosslinking agent were adjusted to obtain Prescription 4' (0.07 g, about 0.30 parts), Prescription 4" (0.09 g, about 0.40 parts), Prescription 4‴ (0.14 g, about 0.60 parts), and Prescription 4⁗ (0.16 g, about 0.70 parts), and the acrylate pressure-sensitive adhesives of corresponding prescriptions were adaptively adjusted (in Prescriptions 4'-4⁗, the amounts of the acrylate pressure-sensitive adhesives were 41.33 g, 40.91 g, 40.49 g, and 41.37 g respectively).

(4) Prescription 5

Dexmedetomidine 0.18 g

Acrylate pressure-sensitive adhesive 41.32 g

Titanium (oxy)acetylacetonate 0.12 g

Propylene glycol 0.30 g

Anhydrous ethanol 5.70 g

N-heptane 6.19 g

The model of the acrylate pressure-sensitive adhesive was Duro-Tak 387-2287; and the preparation method was the same as that of Prescription 2.
The amounts and contents of dexmedetomidine and propylene glycol were unchanged, and the amount and content of the metal chelate crosslinking agent were adjusted to obtain Prescription 5' (0.07 g, about 0.30 parts), Prescription 5" (0.09 g, about 0.40 parts), Prescription 5''' (0.14 g, about 0.60 parts), and Prescription 5'''' (0.16 g, about 0.70 parts), and the acrylate pressure-sensitive adhesives of corresponding prescriptions were adaptively adjusted (in Prescriptions 5'-5'''', the amounts of the acrylate pressure-sensitive adhesives were 41.33 g, 40.91 g, 40.49 g, and 41.37 g respectively).

(5) Prescription 6

Dexmedetomidine 0.18 g
Acrylate pressure-sensitive adhesive 44.85 g
Polybutyl titanate 0.10 g
Propylene glycol 0.32 g
Anhydrous ethanol 6.62 g
N-heptane 1.93 g

The model of the acrylate pressure-sensitive adhesive was Duro-Tak 387-2510, with a solid content of 42.7%. The preparation method of Prescription 6 was the same as that of Prescription 1, except that Scotchpak 9754 backing film was laminated onto the drug-containing adhesive layer.
The amounts and contents of dexmedetomidine and propylene glycol were unchanged, and the amount and content of the metal chelate crosslinking agent were adjusted to obtain Prescription 6' (0.06 g, about 0.30 parts), Prescription 6" (0.08 g, about 0.40 parts), Prescription 6''' (0.12 g, about 0.60 parts), and Prescription 6'''' (0.14 g, about 0.70 parts), and the acrylate pressure-sensitive adhesives of corresponding prescriptions were adaptively adjusted (in Prescriptions 6'-6'''', the amounts of the acrylate pressure-sensitive adhesives were 45.01 g, 44.97 g, 44.87 g, and 44.83 g respectively).

(6) Prescription 7

Dexmedetomidine 0.18 g
Acrylate pressure-sensitive adhesive 44.85 g
Aluminum acetylacetonate 0.10 g
Propylene glycol 0.32 g
Anhydrous ethanol 6.62 g
N-heptane 1.93 g

The model of the acrylate pressure-sensitive adhesive was Duro-Tak 387-2510; and the preparation method was the same as that of Prescription 6.

The amounts and contents of dexmedetomidine and propylene glycol were unchanged, and the amount and content of the metal chelate crosslinking agent were adjusted to obtain Prescription 7' (0.06 g, about 0.30 parts), Prescription 7" (0.08 g, about 0.40 parts), Prescription 7''' (0.12 g, about 0.60 parts), and Prescription 7'''' (0.14 g, about 0.70 parts), and the acrylate pressure-sensitive adhesives of corresponding prescriptions were adaptively adjusted (in Prescriptions 7'-7'''', the amounts of the acrylate pressure-sensitive adhesives were 45.01 g, 44.97 g, 44.87 g, and 44.83 g respectively).

(7) Prescription 8

Dexmedetomidine 0.18 g
Acrylate pressure-sensitive adhesive 44.85 g
Zirconium acetylacetonate 0.10 g
Propylene glycol 0.32 g
Anhydrous ethanol 6.62 g
N-heptane 1.93 g

The model of the acrylate pressure-sensitive adhesive was Duro-Tak387-2510; and the preparation method was the same as that of Prescription 6.

The amounts and contents of dexmedetomidine and propylene glycol were unchanged, and the amount and content of the metal chelate crosslinking agent were adjusted to obtain Prescription 8' (0.06 g, about 0.30 parts), Prescription 8" (0.08 g, about 0.40 parts), Prescription 8''' (0.12 g, about 0.60 parts), and Prescription 8'''' (0.14 g, about 0.70 parts), and the acrylate pressure-sensitive adhesives of corresponding prescriptions were adaptively adjusted (in Prescriptions 8'-8'''', the amounts of the acrylate pressure-sensitive adhesives were 45.01 g, 44.97 g, 44.87 g, and 44.83 g respectively).

(8) Prescription 9

Dexmedetomidine 0.18 g
Acrylate pressure-sensitive adhesive 44.85 g
Titanium (oxy)acetylacetonate 0.10 g
Propylene glycol 0.32 g
Anhydrous ethanol 6.62 g
N-heptane 1.93 g

The model of the acrylate pressure-sensitive adhesive was Duro-Tak387-2510; and the preparation method was the same as that of Prescription 6.

The amounts and contents of dexmedetomidine and propylene glycol were unchanged, and the amount and content of the metal chelate crosslinking agent were adjusted to obtain Prescription 9' (0.06 g, about 0.30 parts), Prescription 9" (0.08 g, about 0.40 parts), Prescription 9''' (0.12 g, about 0.60 parts), and Prescription 9'''' (0.14 g, about 0.70 parts), and the acrylate pressure-sensitive adhesives of corresponding prescriptions were adaptively adjusted (in Prescriptions 9'-9'''', the amounts of the acrylate pressure-sensitive adhesives were 45.01 g, 44.97 g, 44.87 g, and 44.83 g respectively).

2. Test of adhesion performance

According to "General Requirements 0952 Adhesion Determination Method" in Chinese Pharmacopoeia (2020 edition), 8 groups of samples (n=3) of Prescription 2 to Prescription 9 were tested for lasting adhesion and 180°peel strength, and 8 groups of samples of Prescription 2 to Prescription 9 were evaluated for in vivo adhesion performance: the samples were peeled after being pasted for 30 min, and the in vivo adhesion performance was evaluated. The scoring indexes were as follows:

Scoring index:

1: weak in vivo adhesion performance;

2: slight residue of adhesive edge, good adhesive performance;

3: no residue of adhesive edge, good adhesive performance; and

4: causing pain upon removal, including keratinized skin abrasions.

The adhesion performance test results after the above operations are shown in Table 9-Table 16.

3. Experimental results

Table 9 Adhesion performance test results of Prescription 2-Prescription 2''''

| Prescription | Polybutyl titanate (parts) | Average value of lasting adhesion (s) | 180° peel strength (KN/m) | In vivo adhesion performance |
|---|---|---|---|---|
| 2' | 0.30 | 151 | 9.93 | 4 |
| 2" | 0.40 | 631 | 0.10 | 3 |
| 2 | 0.50 | 933 | 0.12 | 3 |
| 2''' | 0.60 | 582 | 0.11 | 3 |
| 2'''' | 0.70 | 98 | 11.12 | 2 |

It can be seen from the above table that with the increase of the amount of polybutyl titanate, the 180° peel strength is decreased first and then increased, and the lasting adhesion is increased greatly within a certain range. When the content of polybutyl titanate is 0.30 parts, slight pain is caused when the sample is torn off; when the content of polybutyl titanate is 0.70 parts, there is a slight adhesive edge residue when the sample is torn off; and when the content of polybutyl titanate is in the range of 0.40-0.60 parts, all the samples tested have good adhesion performance and have no residual trace on the adhesive edge.

Table 10 Adhesion performance test results of Prescription 3-Prescription 3''''

| Prescription | Aluminum acetylacetonate (parts) | Average value of lasting adhesion (s) | 180° peel strength (KN/m) | In vivo adhesion performance |
|---|---|---|---|---|
| 3' | 0.30 | 103 | 6.72 | 4 |
| 3" | 0.40 | 324 | 2.34 | 4 |
| 3 | 0.50 | 876 | 0.18 | 3 |
| 3''' | 0.60 | 678 | 0.15 | 3 |
| 3'''' | 0.70 | 46 | 13.19 | 2 |

It can be seen from the above table that with the increase of the amount of aluminum acetylacetonate, the 180° peel strength is decreased first and then increased, and the lasting adhesion is increased greatly within a certain range. When the content of aluminum acetylacetonate is in the range of 0.30-0.40 parts, slight pain is caused when the sample is torn off; when the content of aluminum acetylacetonate is 0.70 parts, there is a slight adhesive edge residue when the sample is torn off; and when the content of aluminum acetylacetonate is in the range of 0.50-0.60 parts, all the samples tested have good adhesion performance and have no residual trace on the adhesive edge.

Table 11 Adhesion performance test results of Prescription 4-Prescription 4""

| Prescription | Zirconium acetylacetonate (parts) | Average value of lasting adhesion (s) | 180° peel strength (KN/m) | In vivo adhesion performance |
|---|---|---|---|---|
| 4' | 0.30 | 222 | 10.89 | 4 |
| 4" | 0.40 | 456 | 6.08 | 4 |
| 4 | 0.50 | 1022 | 0.09 | 3 |
| 4"' | 0.60 | 786 | 0.16 | 3 |
| 4"" | 0.70 | 91 | 14.52 | 2 |

It can be seen from the above table that with the increase of the amount of zirconium acetylacetonate, the 180° peel strength is decreased first and then increased, and the lasting adhesion is increased greatly within a certain range. When the content of zirconium acetylacetonate is in the range of 0.30-0.40 parts, slight pain is caused when the sample is torn off; when the content of zirconium acetylacetonate is 0.70 parts, there is a slight adhesive edge residue when the sample is torn off; and when the content of zirconium acetylacetonate is in the range of 0.50-0.60 parts, all the samples tested have good adhesion performance and have no residual trace on the adhesive edge.

Table 12 Adhesion performance test results of Prescription 5-Prescription 5""

| Prescription | Titanium (oxy) acetylacetonate (parts) | Average value of lasting adhesion (s) | 180° peel strength (KN/m) | In vivo adhesion performance |
|---|---|---|---|---|
| 5' | 0.30 | 134 | 10.22 | 4 |
| 5" | 0.40 | 297 | 7.16 | 4 |
| 5 | 0.50 | 976 | 0.10 | 3 |
| 5"' | 0.60 | 541 | 0.13 | 3 |
| 5"" | 0.70 | 72 | 12.19 | 2 |

It can be seen from the above table that with the increase of the amount of titanium (oxy)acetylacetonate, the 180° peel strength is decreased first and then increased, and the lasting adhesion is increased greatly within a certain range. When the content of titanium (oxy)acetylacetonate is in the range of 0.30-0.40 parts, slight pain is caused when the sample is torn off; when the content of titanium (oxy)acetylacetonate is 0.70 parts, there is a slight adhesive edge residue when the sample is torn off; and when the content of titanium (oxy)acetylacetonate is in the range of 0.50-0.60 parts, all the samples tested have good adhesion performance and have no residual trace on the adhesive edge.

Table 13 Adhesion performance test results of Prescription 6-Prescription 6""

| Prescription | Polybutyl titanate (parts) | Average value of lasting adhesion (s) | 180° peel strength (KN/m) | In vivo adhesion performance |
|---|---|---|---|---|
| 6' | 0.30 | 165 | 9.86 | 4 |
| 6" | 0.40 | 631 | 0.09 | 3 |
| 6 | 0.50 | 720 | 0.11 | 3 |
| 6"' | 0.60 | 933 | 0.10 | 3 |
| 6"" | 0.70 | 982 | 10.23 | 2 |

It can be seen from the above table that with the increase of the amount of polybutyl titanate, the 180° peel strength is decreased first and then increased, and the lasting adhesion is increased greatly within a certain range. When the content of polybutyl titanate is about 0.30 parts, slight pain is caused when the sample is torn off; when the content of polybutyl titanate is about 0.70 parts, there is a slight adhesive edge residue when the sample is torn off; and when the content of polybutyl titanate is in the range of about 0.40-0.60 parts, all the samples tested have good adhesion performance and have no residual trace on the adhesive edge.

Table 14 Adhesion performance test results of Prescription 7-Prescription 7''''

| Prescription | Aluminum acetylacetonate (parts) | Average value of lasting adhesion (s) | 180° peel strength (KN/m) | In vivo adhesion performance |
|---|---|---|---|---|
| 7' | 0.30 | 96 | 4.75 | 4 |
| 7" | 0.40 | 152 | 1.35 | 4 |
| 7 | 0.50 | 435 | 0.15 | 3 |
| 7''' | 0.60 | 678 | 0.17 | 3 |
| 7'''' | 0.70 | 876 | 7.98 | 2 |

It can be seen from the above table that with the increase of the amount of aluminum acetylacetonate, the 180° peel strength is decreased first and then increased, and the lasting adhesion is increased greatly within a certain range. When the content of aluminum acetylacetonate is in the range of about 0.30-0.40 parts, slight pain is caused when the sample is torn off; when the content of aluminum acetylacetonate is about 0.70 parts, there is a slight adhesive edge residue when the sample is torn off; and when the content of aluminum acetylacetonate is in the range of about 0.50-0.60 parts, all the samples tested have good adhesion performance and have no residual trace on the adhesive edge.

Table 15 Adhesion performance test results of Prescription 8-Prescription 8''''

| Prescription | Zirconium acetylacetonate (parts) | Average value of lasting adhesion (s) | 180° peel strength (KN/m) | In vivo adhesion performance |
|---|---|---|---|---|
| 8' | 0.30 | 225 | 8.91 | 4 |
| 8" | 0.40 | 456 | 5.67 | 4 |
| 8 | 0.50 | 694 | 0.09 | 3 |
| 8''' | 0.60 | 729 | 0.13 | 3 |
| 8'''' | 0.70 | 912 | 10.25 | 2 |

It can be seen from the above table that with the increase of the amount of zirconium acetylacetonate, the 180° peel strength is decreased first and then increased, and the lasting adhesion is increased greatly within a certain range. When the content of zirconium acetylacetonate is in the range of about 0.30-0.40 parts, slight pain is caused when the sample is torn off; when the content of zirconium acetylacetonate is about 0.70 parts, there is a slight adhesive edge residue when the sample is torn off; and when the content of zirconium acetylacetonate is in the range of about 0.50-0.60 parts, all the samples tested have good adhesion performance and have no residual trace on the adhesive edge.

Table 16 Adhesion performance test results of Prescription 9-Prescription 9''''

| Prescription | Titanium (oxy) acetylacetonate (parts) | Average value of lasting adhesion (s) | 180° peel strength (KN/m) | In vivo adhesion performance |
|---|---|---|---|---|
| 9' | 0.30 | 73 | 7.49 | 4 |
| 9" | 0.40 | 297 | 6.54 | 4 |
| 9 | 0.50 | 372 | 0.10 | 3 |
| 9''' | 0.60 | 641 | 0.13 | 3 |
| 9'''' | 0.70 | 935 | 10.28 | 2 |

It can be seen from the above table that with the increase of the amount of titanium (oxy)acetylacetonate, the 180° peel strength is decreased first and then increased, and the lasting adhesion is increased greatly within a certain range. When the content of titanium (oxy)acetylacetonate is in the range of about 0.30-0.40 parts, slight pain is caused when the sample is torn off; when the content of titanium (oxy)acetylacetonate is about 0.70 parts, there is a slight adhesive edge residue when the sample is torn off; and when the content of titanium (oxy)acetylacetonate is in the range of about 0.50-0.60 parts, all the samples tested have good adhesion performance and have no residual trace on the adhesive edge.

To sum up, the addition of metal chelate crosslinking agent can improve the adhesion performance of the dexmedetomidine transdermal patch of the present application. Through the comprehensive evaluation of the three tests of lasting adhesion, 180° peel strength, and in vivo adhesion performance, it can be seen that the amount of the metal chelate crosslinking agent in the transdermal patch of the present application is 0.30-0.70 parts, preferably, 0.40-0.60 parts, and more preferably, 0.50 parts.

**Example 4 Prescription 10**

> Dexmedetomidine 0.18 g
> Acrylate pressure-sensitive adhesive 32.40 g
> Polybutyl titanate 0.09 g
> Propylene glycol 0.3 g
> Anhydrous ethanol 5.70 g
> N-heptane 1.33 g

The model of the acrylate pressure-sensitive adhesive was Duro-Tak 387-2287, with a solid content of 53.8%.
The preparation method of Prescription 10 was the same as that of Prescription 1, except that Scotchpak 9709 was used for coating and Scotchpak 9723 was used for lamination.

**Example 5 Prescription 11**

> Dexmedetomidine 0.09 g
> Acrylate pressure-sensitive adhesive 18.71 g
> Polybutyl titanate 0.05 g
> Propylene glycol 0.15 g
> Anhydrous ethanol 3.04 g
> N-heptane 0.96 g

The model of the acrylate pressure-sensitive adhesive was Duro-Tak 387-2510, with a solid content of 42.7%.
The preparation method of Prescription 11 was the same as that of Prescription 1, except that Scotchpak 9709 was used for coating and Scotchpak 9723 was used for lamination.

**Example 6 Prescription 12**

> Dexmedetomidine 0.14 g
>
> Acrylate pressure-sensitive adhesive 32.66 g
>
> Aluminum acetylacetonate 0.07 g
>
> Propylene glycol 0.24 g
>
> Anhydrous ethanol 7.64 g
>
> N-heptane 2.50 g

The model of the acrylate pressure-sensitive adhesive was Duro-Tak 387-2510, with a solid content of 42.7%.

The preparation method of Prescription 8 was the same as that of Prescription 1, except that backing film Scotchpak 9745 was laminated onto the drug-containing adhesive layer.

**Example 7 Prescription 13**

Dexmedetomidine 0.18 g

Acrylate pressure-sensitive adhesive 40.82 g

Polybutyl titanate 0.09 g

Propylene glycol 0.30 g

Anhydrous ethanol 5.70 g

N-heptane 1.44 g

The model of the acrylate pressure-sensitive adhesive was Duro-Tak 387-2510, with a solid content of 42.7%.

The preparation method of Prescription 9 was the same as that of Prescription 1, except that Scotchpak 9709 was selected as the release film; Scotchpak 9745 was selected as the backing film; and at the time of coating, the thickness of the drug-containing layer was adjusted to 50 $\mu$m.

**Example 8 Prescription 14**

Dexmedetomidine 0.03 g

Pressure-sensitive adhesive 23.00 g

Aluminum acetylacetonate 0.03 g

Propylene glycol 0.04 g

Anhydrous ethanol 2.50 g

N-heptane 1.25 g

The model of the acrylate pressure-sensitive adhesive was Duro-Tak 387-2510, with a solid content of 42.7%.

The preparation method of Prescription 10 was the same as that of Prescription 1, except that Scotchpak 9709 was selected as the release film; Scotchpak 9745 was selected as the backing film; and at the time of coating, the thickness of the drug-containing layer was adjusted to 50 $\mu$m.

**Example 9 Prescription 15**

Dexmedetomidine 0.09 g

Pressure-sensitive adhesive 28.45 g

Polybutyl titanate 0.04 g

Propylene glycol 0.15 g

Anhydrous ethanol 5.70 g

N-heptane 1.44 g

The model of the acrylate pressure-sensitive adhesive was Duro-Tak 387-2510, with a solid content of 42.7%.
The preparation method of Prescription 11 was the same as that of Prescription 1, except that Scotchpak 9709 was selected as the release film; Scotchpak 9745 was selected as the backing film; and at the time of coating, the thickness of the drug-containing layer was adjusted to 50 µm.

**Example 10 Prescription 16**

Dexmedetomidine 0.36 g

Pressure-sensitive adhesive 43.50 g

Aluminum acetylacetonate 0.16 g

Propylene glycol 0.84 g

Anhydrous ethanol 5.70 g

N-heptane 6.80 g

The model of the acrylate pressure-sensitive adhesive was Duro-Tak 387-2510, with a solid content of 42.7%.
The preparation method of Prescription 12 was the same as that of Prescription 1, except that Scotchpak 9709 was selected as the release film; Scotchpak 9745 was selected as the backing film; and at the time of coating, the thickness of the drug-containing layer was adjusted to 50 µm.

**Example 11 Prescription 17**

Dexmedetomidine 0.72 g

Pressure-sensitive adhesive 49.20 g

Polybutyl titanate 0.30 g

Propylene glycol 1.92 g

Anhydrous ethanol 5.60 g

N-heptane 9.50 g

The model of the acrylate pressure-sensitive adhesive was Duro-Tak 387-2510, with a solid content of 42.7%.
The preparation method of Prescription 13 was the same as that of Prescription 1, except that Scotchpak 9709 was selected as the release film; Scotchpak 9745 was selected as the backing film; and at the time of coating, the thickness of the drug-containing layer was adjusted to 50 µm.

**Example 11 Investigation of coating thickness**

**1. Method**

Samples prepared by Prescription 13 (D=14 mm) were used, the thickness of the drug-containing layer was adjusted to 25 µm, 50 µm, and 100 µm, and the samples were divided into three groups.
The anti-adhesion layer was removed from the prepared transdermal patch, and the transdermal patch was applied to the surface of pigskin, and gently pressed with fingers to ensure good adhesion between the pigskin and the patch. The pigskin was then attached between a Franz diffusion cell provided with a rotor and a supply chamber, with the backing layer in contact with air, and fixed with an iron clip. 9 mL receiving medium was measured with a pipette and entered a receiving chamber, and magnetic stirrers were added for stirring at a constant temperature (32±0.5°C) and a constant speed (180 r/min). A 1 mL syringe was used for taking 0.8 mL sample at predetermined time points, and at the same time, a blank receiving fluid at the same temperature and the same amount was supplemented. The samples taken out were immediately sent to high performance liquid chromatography for determination of content. The results of in vitro transdermal diffusion were shown in the following table.

**2. Experimental results**

Table 17 Cumulative diffused amount per unit area of samples with different thicknesses

| Diffused amount per unit area ($\mu$g/cm$^2$) | | | |
|---|---|---|---|
| Time (h) | Thickness | | |
| | 100 $\mu$m | 50 $\mu$m | 25 $\mu$m |
| 4 | 1.19 | 1.65 | 1.55 |
| 8 | 5.31 | 5.51 | 4.82 |
| 12 | 10.84 | 9.91 | 7.93 |
| 24 | 30.48 | 22.31 | 15.07 |
| 36 | 46.24 | 29.85 | 17.87 |
| 48 | 56.03 | 33.73 | 19.11 |
| 60 | 64.04 | 35.44 | 19.58 |
| 72 | 68.05 | 36.96 | 19.77 |
| 96 | 72.33 | 35.78 | 20.00 |
| 120 | 74.24 | 35.44 | 20.13 |

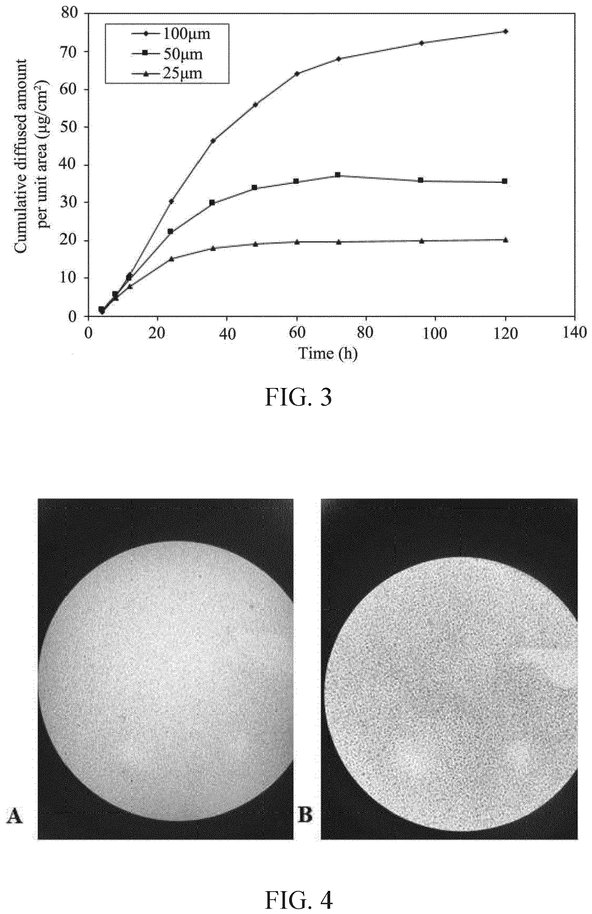

Specifically, the in vitro transdermal diffusion curves of patches with different thicknesses are shown in FIG. 3. It can be seen from FIG. 3 that the thickness of the dexmedetomidine skeleton transdermal patch is related to the duration of action, and increasing the thickness of the transdermal patch can prolong the duration of action of the transdermal patch. The dexmedetomidine transdermal patch with a thickness of 25 $\mu$m reaches the drug release plateau at 48 h; the patch with a thickness of 50 $\mu$m reaches the drug release plateau at 72 h; and the patch with a thickness of 100 $\mu$m reaches the drug release plateau at 120 h. Therefore, the dexmedetomidine patch formulation with a thickness of 25 $\mu$m to 100 $\mu$m can achieve sustained release for 2-5 days.

**Test Example 1 Skin response test of transdermal patch**

Evaluation indexes:

Skin response levels include:

Level 4: erythema, blistering, and bulla formation;

Level 3: erythema, blistering, and no bullae;

Level 2: erythema all over the patch area; no blisters;

Level 1: mild erythema in an area less than the whole patch area; and

Level 0: minimum or no response at the patch site.

Two samples of each of Prescriptions 1-17 of the present application were applied to 18 rats with back hair removed for 24 h. The results showed that the products of Prescriptions 1-17 had no skin irritation, at Level 0.

**Test Example 2 Study on stability of preparation**

Round patches of samples (n=3) of the above Prescription 1-Prescription 17 were subjected to stress testing for investigation of related substances, wherein average values were taken, the content limits of impurities were as follows, and the investigation results were as follows.

Table 18 Test results of related substances in the investigation of influencing factors in prescriptions

| Prescription | 0 days | | High temperature 60 °C (10 days) | | High temperature 60 °C (30 days) | | Acceleration 1 month | |
|---|---|---|---|---|---|---|---|---|
| | Maximum unknown single impurity (large unknown single impurity) | Total impurities (unknown single impurity) | Maximum unknown single impurity (large unknown single impurity) | Total impurities (unknown single impurity) | Maximum unknown single impurity (large unknown single impurity) | Total impurities (unknown single impurity) | Maximum unknown single impurity (large unknown single impurity) | Total impurities (unknown single impurity) |
| Prescription 1 | 0.2% | 0.4% | 0.2% | 1.0 % | 0.5% | 2.0% | 0.2% | 0.1% |
| Prescription 2 | 0.3% | 0.4% | 0.3% | 1.0 % | 0.3% | 2.0% | 0.1% | 0.1% |
| Prescription 3 | 0.2% | 0.3% | 0.2% | 0.9% | 0.4% | 2.0% | 0.1% | 0.1% |
| Prescription 4 | 0.2% | 0.6% | 0.2% | 0.9% | 0.4% | 1.9% | 0.1% | 0.1% |
| Prescription 5 | 0.4% | 0.5% | 0.2% | 0.8% | 0.4% | 2.0% | 0.2% | 0.2% |
| Prescription 6 | 0.1% | 0.1% | ND | ND | 0.1% | 0.1% | 0.1% | 0.1% |
| Prescription 7 | 0.1% | 0.2% | 0.1% | 0.1% | 0.1% | 0.1% | 0.3% | 0.3% |
| Prescription 8 | 0.1% | 0.2% | ND | ND | 0.1% | 0.2% | 0.2% | 0.2% |
| Prescription 9 | 0.1% | 0.2% | ND | ND | 0.1% | 0.1% | 0.2% | 0.2% |

| | | | | | | | | |
|---|---|---|---|---|---|---|---|---|
| Prescription 10 | 0.2% | 0.2% | 0.3% | 0.3% | 0.3% | 0.5% | 0.3% | 0.4% |
| Prescription 11 | 0.1% | 0.1% | 0.1% | 0.1% | 0.1% | 0.2% | 0.2% | 0.2% |
| Prescription 12 | 0.2% | 0.6% | 0.2% | 0.9% | 0.3% | 1.9% | 0.2% | 0.2% |
| Prescription 13 | 0.1% | 0.3% | 0.2% | 0.7% | 0.3% | 1.9% | 0.1% | 0.1% |
| Prescription 14 | 0.2% | 0.6% | 0.2% | 0.9% | 0.5% | 1.9% | 0.1% | 0.1% |
| Prescription 15 | 0.4% | 0.7% | 0.4% | 0.6% | 0.4% | 2.0% | 0.2% | 0.2% |
| Prescription 16 | 0.3% | 0.3% | 0.2% | 0.8% | 0.3% | 2.0% | 0.1% | 0.1% |
| Prescription 17 | 0.1% | 0.4% | 0.3% | 0.8% | 0.4% | 1.8% | 0.2% | 0.2% |

As can be seen from the above table, the storage of the dexmedetomidine transdermal patch of the present application is stable under high temperature conditions for 10 days, high temperature conditions for 30 days, and acceleration conditions.

**Test Example 3 Study on crystallization of dexmedetomidine**

Samples prepared according to Prescription 13 were used for observation of the long-term crystallization of the dexmedetomidine transdermal patch of the present application to confirm whether crystallization of dexmedetomidine would occur in the prescription.

The crystallization observation results after the samples were stored for 6 months are shown in FIG. 4 (A is $10\times$ imaging and B is $40\times$ imaging). By observing the crystallization status with polarizing microscope, it could be seen that the dexmedetomidine transdermal patch prepared in the present application did not produce crystallization during 6 months of long-term storage.

**Test Example 4 Monitoring of amount of voluntary activity of mice**

1. Method

24 h after hair removal, the mice were placed in an activity instrument for adaptive training. 10 min later, activity within 5 min was monitored, and the result was recorded as 0h activity. The drug-containing patch prepared by Prescription 13 was given according to the mouse pharmacodynamic experimental scheme in Table 19, the mice were placed in the activity instrument for monitoring of the mouse activity after administration. Monitoring of mouse activity was continued after the monitoring time in a later stage was reached, and the monitoring time points were 1 h, 3 h, 6 h, 8 h, 12 h, and 24 h.

After 24 h monitoring was ended, the patch on the back of the mouse was removed, and the activity of the mouse continued to be monitored at 27 h and 30 h, using the same monitoring method as described above.

Evaluation index: recording the number of activities within 5 min.

Table 19 Experimental scheme of pharmacodynamics in mice

| Group | Dose (μg/mouse) | Route of administration | Number of animals (mice) |
|---|---|---|---|
| Blank control group | NA | Administration via skin | 8 |
| Test product dose group 1 | 4.2 | Administration via skin | 8 |
| Test product dose group 2 | 18.5 | Administration via skin | 8 |
| Test product dose group 3 | 37.1 | Administration via skin | 8 |
| Test product dose group 4 | 53.3 | Administration via skin | 8 |

2. Experimental results

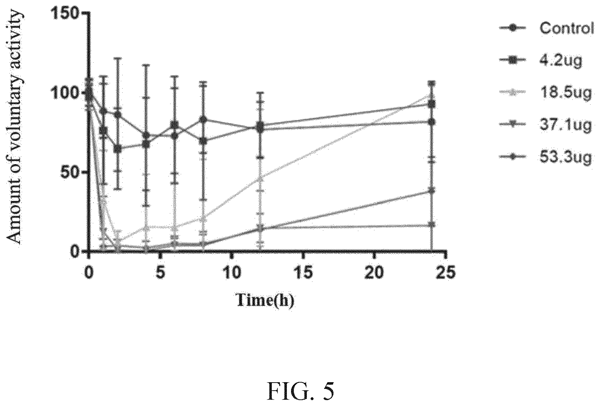

The data of amount of voluntary activity of mice is shown in FIG. 5. Compared with the blank control, the dexmedetomidine transdermal patch can significantly reduce the volume of activity of mice, showing an obvious dose-effect relationship, indicating that dexmedetomidine has a significant effect on sleep improvement.

**Test Example 5 Electroencephalography and electromyography in rats**

1. Grouping

Before administration, the experimental animals were weighed and randomly grouped according to their weights, as follows:

Table 20 Experimental grouping for electroencephalography and electromyography in rats

| Group | Number of animals | Substance to be tested | Administration scheme (dose \| mode of administration \| frequency) |
|---|---|---|---|
| Normal group (Sham group) | 8 | Blank patch | Single application to the back for 72 h |
| Postoperative pain model group (Vehicle group) | 8 | Blank patch | Single application to the back for 72 h |
| Low dose group | 8 | Drug-containing patch prepared by Prescription 13 | 0.88 mg/kg, single application to the back for 72 h |
| Medium dose group | 8 | Drug-containing patch prepared by Prescription 13 | 1.77 mg/kg, single application to the back for 72 h |
| High dose group | 8 | Drug-containing patch prepared by Prescription 13 | 2.65 mg/kg, single application to the back for 72 h |

2. Method

SD rats (SPF grade, male) were exposed to 12 h alternating light and dark for 7 days (light was turned off at 7:00; light was turn on at 19:00). The animals were anesthetized with Zoletil (i.p., 20 mg/kg) combined with xylazine (i.p., 8 mg/kg) on the day of the experiment. After anesthetization, they were fixed by stereotaxic apparatus. After the head was dehaired and sterilized, the skin of the head was cut, the four corners were clamped with hemostatic forceps, to fully expose the skull, and the periosteum was peeled off, and was wiped with dry absorbent cotton until the surface was dry and clean. The skull was drilled and electrodes were embedded. Two electromyographic electrodes were inserted into the neck muscles in parallel, with both ends secured with sutures to avoid mutual contact at ends. A reference electrode was likewise inserted into the contralateral neck muscle and fixed. An implant was then placed under the skin, and the surgical wound was sutured and disinfected. After surgery, the rat was carefully placed in a clean recovery cage, in a lateral position to ensure an open airway. The rat was reared in a single cage and placed in a shielded recovery room, with 12 h automatic alternation of light and dark: turning off the light at 7:00, and turning on the light at 19:00. The animals were given 3 days care after surgery. The surgical incision was treated locally with Cephradine powder, gentamicin was given subcutaneously at 4-8 mg/kg, and meloxicam was injected subcutaneously at 0.1 ml/rat for 3 consecutive days. The experiment was conducted after 7-10 days of recovery, and the animals were randomly grouped according to their body weight. After grouping, EEG and EMG were continuously monitored for 24 h as baseline signals.

Except the Sham group, skin and fascia incisions with a longitudinal length of about 2 cm were made on the back and pelma of the rats in each group, and then sutured. After all the animals were modeled, the rats in each group were dehaired with an animal shaver at the site of drug administration, and given corresponding drugs (the patch was located on the back and fixed on the skin to prevent the patch from falling off halfway and causing failure of the drug administration). Recording lasted for 72 h. EEG and EMG signals of the rats in this period were monitored, and the structural changes of postoperative Wake, light sleep or REM sleep, and deep sleep or NREM sleep were analyzed.

3. Data analysis

The original data was collected by Ponemah software of DSI system and analyzed by NeuroScore software. The experimental data was expressed by mean±standard deviation (Mean±S.E.M.), and was statistically analyzed by Graph Pad Prism 7.0 software and using Two-way ANOVA and One-way ANOVA. $P<0.05$ indicated having significant differences, $P<0.01$ indicated having very significant differences, and $P<0.001$ indicated having extremely significant differences.

4. Experimental results

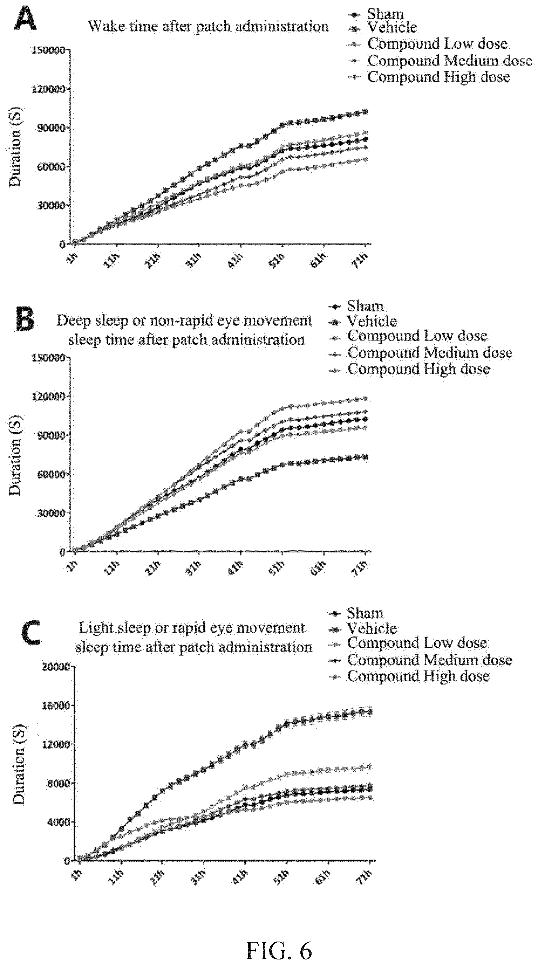

As shown in FIG. 6, A: a graph of Wake time cumulative trend of each group within each time node in the rat sleep structure within 72 h after administration of a patch; B: a graph of NREM time cumulative trend of each group in each time node within 72 h after administration of a patch; C: a graph of REM time cumulative trend of each group in each time node within 72 h after administration of a patch; N=8.

Experimental data in FIG. 6 shows that:
Compared with the normal group, with the increase of the dose of dexmedetomidine, the Wake time of the rats in the drug-containing group within 72 h decreased gradually, and there was no significant difference in the Wake time within 72 h between the low dose group and the normal group; and compared with the postoperative model group, the Wake time of the rats in the drug-containing patch group within 72 h decreased significantly.

Compared with the normal group, with the increase of the dose of dexmedetomidine, the slow-wave sleep time of the rats in the drug-containing group within 72 h was prolonged gradually, and there was no significant difference in the slow-wave sleep time within 72 h between the low dose group and the normal group; and compared with the postoperative model group, the slow-wave sleep time of the rats in the drug-containing patch group within 72 h was significantly prolonged.

Generally speaking, the dexmedetomidine transdermal patch of the present application can effectively prolong the NREM sleep time, reduce the REM sleep time of rats after surgery, and can effectively improve the sleep quality. The transdermal patch at a high dose has a great influence on the sleep duration of SD rats, so that the proportion of awake period is significantly shortened, which affects the normal circadian rhythm of animals to a certain extent. The transdermal patch at medium and high doses can continuously and effectively improve the postoperative sleep disorder and promote sleep quality of rats for 72 h, and the duration of the effect is longer than that at a low dose (0.88 mg/kg). However, the low dose group can significantly improve the sleep quality, and does not alter the circadian rhythm as in the medium and high dose group. The low dose group is substantially the same as the normal group in terms of the total duration of slow-wave sleep and the total Wake duration within 72 h.

**Comparative Example 1**

1. Preparation of prescription

Comparative example of the present application was prepared according to preparation 33 in the reference document CN201480059798.5, and the specific prescription was as follows:

Dexmedetomidine 0.18 g

Levulinic acid 0.11 g

Acrylate pressure-sensitive adhesive 32.92 g

The model of the acrylate pressure-sensitive adhesive was Duro-Tak 87-2287, with a solid content of 53.8%.
The dexmedetomidine transdermal patch of Comparative Example 1 of the present application was prepared according to the preparation method of the dexmedetomidine transdermal composition described in the reference document: the preparation was prepared by mixing dexmedetomidine and a pressure-sensitive adhesive in an organic solvent, followed by mixing. Once a homogeneous mixture was formed, the solution was poured on a release liner and dried at 60°C-80°C for 10-90 minutes. Then, the single-layered adhesive thin film was laminated to the PET backing, followed by cutting into a desired size and putting into a bag. In this comparative example, levulinic acid was added to the adhesive composition.
The solvent contained in the pressure-sensitive adhesive and the organic solvent added in the preparation process were removed during the drying process.

2. Transdermal diffusion test in vitro

Three patches were taken from the patches prepared in each of Example 7 and Example 4 of the present application and Comparative Example 1, with the release film layer removed for in vitro transdermal diffusion test. The test method was the same as that in Example 12.

3. Experimental results

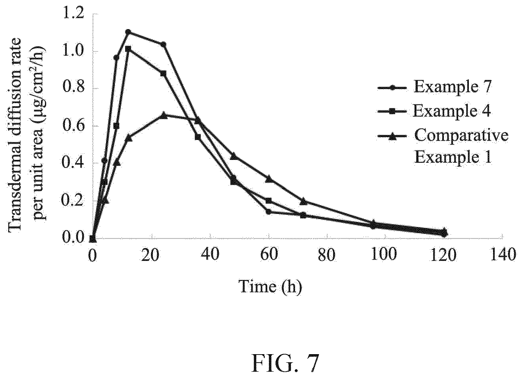

The in vitro transdermal diffusion curves of the three groups of transdermal patches are shown in FIG. 7. As can be seen from FIG. 7, the overall trend of the diffusion rate of the three groups of transdermal patches is that Example 7 is superior to Example 4 which is superior to Comparative Example 1, the transdermal diffusion rate per unit area of the dexmedetomidine transdermal patch prepared in Example 7 reaches the maximum at 12 h, which is 1.1 pg/cm$^2$*h, about 2 times of that in Comparative Example 1, and the patch prepared in Comparative Example 1 reaches the maximum transdermal diffusion rate per unit area at 24 h. On the whole, the in vitro diffusion rate of the transdermal patch prepared in the present application is better than that in the reference document 1.
The foregoing is only preferred embodiments of the present application, and it should be noted that, for those of ordinary skills in the art, several improvements and modifications may be made without departing from the principles of the present application, and such improvements and modifications are also to be considered as the scope of protection of the present application.

**Claims**

1. A dexmedetomidine transdermal composition, wherein the composition comprises dexmedetomidine, propylene glycol, a metal chelate crosslinking agent, and a pressure-sensitive adhesive.

2. The dexmedetomidine transdermal composition according to claim 1, wherein in parts by weight, in the composition,

Dexmedetomidine 0.30-3.00 parts;
Propylene glycol 0.40-8.00 parts;
Metal chelate crosslinking agent 0.30-1.25 parts;
Pressure-sensitive adhesive 50.00-99.00 parts;
the mass ratio of propylene glycol to dexmedetomidine is (4:3)-(8:3).

3. The dexmedetomidine transdermal composition according to claim 2, wherein the mass ratio of propylene glycol to dexmedetomidine in the composition is (5:3)-(7:3); or the mass ratio of propylene glycol to dexmedetomidine in the composition is 5:3.

4. The dexmedetomidine transdermal composition according to claim 2 or 3, wherein the mass ratio of the pressure-sensitive adhesive to the metal chelate crosslinking agent is (70:1)-(310:1).

5. The dexmedetomidine transdermal composition according to claim 4, wherein the mass ratio of the pressure-sensitive adhesive to the metal chelate crosslinking agent is (70:1)-(160:1), (160:1)-(220:1), or (220:1)-(310:1); or (160:1)-(220:1), (160:1)-(210:1), (160:1)-(200:1), or (190:1)-(220:1); or 160:1, 170:1, 180:1, 190:1, 200:1, 210:1, or 220:1.

6. The dexmedetomidine transdermal composition according to any one of claims 1 to 5, wherein the metal chelate crosslinking agent is selected from one or more of aluminum acetylacetonate, zirconium acetylacetonate, titanium acetylacetonate, and polybutyl titanate.

7. The dexmedetomidine transdermal composition according to any one of claims 1 to 6, wherein the pressure-sensitive adhesive is selected from one or more of an acrylate pressure-sensitive adhesive, a polyisobutylene pressure-sensitive adhesive, a silicone pressure-sensitive adhesive, a styrene-isoprene-styrene hot melt pressure-sensitive adhesive, and a vinyl acetate copolymer.

8. A dexmedetomidine transdermal patch, wherein the dexmedetomidine transdermal patch comprises in the following order a backing layer, an adhesive layer, and an anti-adhesion release film layer; and the adhesive layer is formed by the dexmedetomidine transdermal composition according to any one of claims 1 to 7.

9. The dexmedetomidine transdermal patch according to claim 8, wherein the adhesive layer has a thickness of 25 $\mu$m-100 $\mu$m.

10. A method for preparing the dexmedetomidine transdermal patch according to claim 8 or 9, comprising the steps of:

dissolving a metal chelate crosslinking agent in a solvent to obtain a metal chelate crosslinking agent solution;
mixing a pressure-sensitive adhesive with the metal chelate crosslinking agent solution to obtain a blank matrix solution;
mixing dexmedetomidine, propylene glycol, and a solvent to obtain a dexmedetomidine solution;
mixing the dexmedetomidine solution with the blank matrix solution, followed by stirring and standing, to obtain a drug-containing matrix solution;
coating the drug-containing matrix solution onto an anti-adhesion release film layer, and drying to obtain a composite layer of an adhesive layer and the anti-adhesion release film layer; and
laminating a backing layer onto the adhesive layer.

11. The method according to claim 10, wherein the solvent is selected from one of anhydrous ethanol and n-heptane, or a mixture thereof.

12. Use of the dexmedetomidine transdermal composition according to any one of claims 1 to 7 or the dexmedetomidine transdermal patch according to any one of claims 8 to 9 in the preparation of a pharmaceutical preparation for improving a sleep disorder.

13. The use according to claim 12, wherein the sleep disorder is one or more of a perioperative sleep disorder, a senile sleep disorder, and a traumatic sleep disorder.

14. A method for improving a sleep disorder in a subject, the method comprising administering the dexmedetomidine transdermal composition according to any one of claims 1 to 7 or the dexmedetomidine transdermal patch according to any one of claims 8 to 9 to a subject in need thereof.

**Dosing 2**
④ Dexmedetomidine
⑤ Propylene glycol
⑥ Solvent

**Dosing 1**
① Pressure-
sensitive
adhesive
② Metal chelate
crosslinking
agent
③ Solvent

Mixing 1 → Mixing 2 → Coating

Drying

Punching and packaging ← Laminating ← Drying

FIG. 1

A、

First layer: backing layer
Second layer: drug storage skeleton layer
Third layer: release film layer

Dimple          Dimple

B、

FIG. 2

FIG. 3

FIG. 4

FIG. 5

FIG. 6

FIG. 7

<div align="center">

**INTERNATIONAL SEARCH REPORT**

</div>

| | International application No. |
|---|---|
| | **PCT/CN2022/113953** |

**A.  CLASSIFICATION OF SUBJECT MATTER**

A61K 9/70(2006.01)i;  A61K 31/4174(2006.01)i;  A61K 47/10(2006.01)i;  A61K 47/12(2006.01)i;  A61K 47/32(2006.01)i;  A61P 25/20(2006.01)i

According to International Patent Classification (IPC) or to both national classification and IPC

**B.  FIELDS SEARCHED**

Minimum documentation searched (classification system followed by classification symbols)

A61K A61P

Documentation searched other than minimum documentation to the extent that such documents are included in the fields searched

Electronic data base consulted during the international search (name of data base and, where practicable, search terms used)

CNTXT; ENTXTC; VCN; CJFD; DWPI; CNKI; WFNPL; NPTXT; NPABS; Web of Science; 读秀, DUXIU: 美托咪啶, 美托咪定, 美托嘧啶, 压敏胶, 螯合物, 交联剂, 丙二醇, 经皮, 透皮, 贴剂, 乙酰丙酮酸, 聚钛酸丁酯, Medetomidine, Domtor, Dexmedetomidine, MPV 1440, Pressure sensitive adhesive, chelate, cross-linking agent, propylene glycol, transdermal, patch, acetylpyruvate, polybutyl titanate

**C.  DOCUMENTS CONSIDERED TO BE RELEVANT**

| Category* | Citation of document, with indication, where appropriate, of the relevant passages | Relevant to claim No. |
|---|---|---|
| X | US 2016310441 A1 (MARUISHI PHARMACEUTICAL CO., LTD. et al.) 27 October 2016 (2016-10-27) <br> description, embodiment 2, test example 1, and paragraph [0043] | 1-13 |
| Y | CN 110585173 A (TEIKOKU PHARMA USA INC.) 20 December 2019 (2019-12-20) <br> description, paragraphs [0033], [0050], [0051], [0059], [0060], and [0085] | 1-13 |
| Y | 李先福等 (LI, Xianfu et al.). "盐酸右美托咪定凝胶剂的原辅料相容性研究 (Compatibility of Raw Material and Excipients in Dexmetomidine Hydrochloride Gel)" <br> 国际药学研究杂志 (Journal of International Pharmaceutical Research), <br> Vol. 46, No. 11, 30 November 2019 (2019-11-30), <br> pp. 867-872 | 1-13 |
| Y | 张肖玲等 (ZHANG, Xiaoling et al.). "醋酸烯诺孕酮透皮贴剂的制备及不同促渗剂对其促渗作用考察 (Preparation of Nestorone Transdermal Patches and Study on the Effect of Different Permeation Enhancer on It)" <br> 中国药房 (China Pharmacy), Vol. 24, No. 17, 31 December 2013 (2013-12-31), <br> pp. 1587-1590 | 1-13 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **09 October 2022** | **21 October 2022** |

| Name and mailing address of the ISA/CN | Authorized officer |
|---|---|
| **China National Intellectual Property Administration (ISA/ CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2022/113953** |

| C. | DOCUMENTS CONSIDERED TO BE RELEVANT | |
| --- | --- | --- |
| Category* | Citation of document, with indication, where appropriate, of the relevant passages | Relevant to claim No. |
| A | 魏小藏等 (WEI, Xiaocang et al.). "甲巯咪唑水凝胶贴剂的制备和体外透皮性能 (Preparation of Thiamazole Hydrogel Patch and its In Vitro Percutaneous Permeability)" 中国医院药学杂志 (Chinese Journal of Hospital Pharmacy), Vol. 28, No. 05, 31 March 2008 (2008-03-31), pp. 348-351 | 1-13 |
| A | 武志富等 (WU, Zhifu et al.). "甘氨酸铝螯合物的表征与结构分析 (Synthesis and Structure Analysis of Aluminium Glycinate Monohydrate)" 化学世界 (Chemical World), No. 2,, 31 December 2011 (2011-12-31), pp. 92-96 | 1-13 |

Form PCT/ISA/210 (second sheet) (January 2015)

**INTERNATIONAL SEARCH REPORT**

International application No.

**PCT/CN2022/113953**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **14**
because they relate to subject matter not required to be searched by this Authority, namely:

[1] The method involved in claim 14 is a method for the treatment of diseases, which is implemented on a living human or animal body and directly aims at treating diseases, belongs to a method for treating a human or animal body, and belongs to the cases listed in PCT Rule 39.1(iv) for which no international search is required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

<div style="text-align:center">

**INTERNATIONAL SEARCH REPORT**
Information on patent family members

</div>

| International application No. |
| --- |
| **PCT/CN2022/113953** |

| Patent document cited in search report | | | Publication date (day/month/year) | Patent family member(s) | | | Publication date (day/month/year) |
| --- | --- | --- | --- | --- | --- | --- | --- |
| US | 2016310441 | A1 | 27 October 2016 | WO | 2015093503 | A1 | 25 June 2015 |
| | | | | TW | 201609200 | A | 16 March 2016 |
| | | | | EP | 3087985 | A1 | 02 November 2016 |
| | | | | JP | WO2015093503 | A1 | 23 March 2017 |
| CN | 110585173 | A | 20 December 2019 | BR | 112016006842 | A2 | 01 August 2017 |
| | | | | RU | 2018105761 | A | 26 February 2019 |
| | | | | US | 2015098980 | A1 | 09 April 2015 |
| | | | | RU | 2016109449 | A | 13 November 2017 |
| | | | | TW | 201840306 | A | 16 November 2018 |
| | | | | KR | 20180017224 | A | 20 February 2018 |
| | | | | TW | 201526926 | A | 16 July 2015 |
| | | | | ES | 2856748 | T3 | 28 September 2021 |
| | | | | KR | 20160065200 | A | 08 June 2016 |
| | | | | EP | 3054932 | A1 | 17 August 2016 |
| | | | | CN | 105764494 | A | 13 July 2016 |
| | | | | CA | 2924231 | A1 | 16 April 2015 |
| | | | | JP | 2016532642 | A | 20 October 2016 |
| | | | | WO | 2015054058 | A1 | 16 April 2015 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110969146

- CN 201480059798